# EUROPEAN PATENT SPECIFICATION

(11) **EP 0 981 626 B1**
(45) Date of publication and mention of the grant of the patent: **17.12.2008**
(21) Application number: 98907029.7
(22) Date of filing: 23.02.1998
(51) Int. Cl.: C12N 15/53, C12N 15/70, C12N 9/04, C12N 1/15, C12Q 1/32, C12R 1/645

(54) **FUNGICIDAL TEST METHOD**
TESTVERFAHREN FÜR FUNGIZIDE
METHODE D'ESSAI DE FONGICIDES

(30) Priority: 22.02.1997 GB 9703739
(43) Date of publication of application: 01.03.2000
(73) Proprietor: Bayer CropScience Limited, 230 Cambridge Science Park Milton Road Cambridge BC4 0WB (GB)
(72) Inventor: FOSTER, Stephen, George, Essex CB10 1XL (GB); COOLEY, Russell, Neil, Essex CB10 1XL (GB)
(74) Representative: Monconduit, Hervé
(86) International application number: PCT/GB1998/000566
(87) International publication number: WO 1998/037203

(56) References cited:
- LI Q. ET AL: "The leu-1 gene of Neurospora crassa: Nucleotide and deduced amino acid sequence comparisons." GENE, (1993) 136/1-2 (301-305). ISSN: 0378-1119 CODEN: GENED6, XP002070834
- BYUN, M. O. ET AL: "Transformation of the beta-isopropylmalate dehydrogenase gene of Flammulina velutipes into Pleurotus florida" KOREAN JOURNAL OF MYCOLOGY, (1989) VOL. 17, NO. 1, PP. 27-30. 9 REF., XP002070835
- PATENT ABSTRACTS OF JAPAN vol. 013, no. 286 (C-613), 29 June 1989 & JP 01 080295 A (TAKEDA CHEM IND LTD), 27 March 1989,
- NEWTON A. AND CATEN C.: "Auxotrophic mutants of Septoria nodorum isoalted by direct screening and by selection for resistance to chlorate" TRANSACTIONS OF THE BRITISH MYCOLOGICAL SOCIETY, vol. 90, no. 2, 1988, pages 199-207, XP002070836

## Description

### Field of the Invention

This invention relates to the surprising observation that the enzyme 3-isopropylmalate dehydrogenase is essential for the pathogenicity of the plant pathogenic fungus *Septoria nodorum.* This invention further relates to a method for identifying fungicides and the use of inhibitors of 3-isopropylmalate dehydrogenase as fungicides.

### Prior Art

It has been demonstrated that some induced biochemical mutants of plant pathogenic fungi such as *Venturia inaequalis* (a hemi-biotroph) are no longer pathogenic (Boone et al, 1957, American Journal of Botany 44, 791 and Kline et al ibid, 797). In this, and in the other examples, these biochemical mutants have not been further analysed to define the exact point of the biochemical lesion. The isolation and expression of genes from one organism to another is common practice to those skilled in the art. Gene disruption is well known to those skilled in the art and has been recently reviewed by Oliver R J and Osbourne, A E (1995), Microbiology, 141, 191.

### Disclosure of the Invention

We have demonstrated that a mutant of the plant pathogenic fungus *Septoria nodorum* which is unable to synthesise the amino acid leucine is non-pathogenic. We also demonstrate that a defective enzyme, 3-isopropylmalate dehydrogenase (EC 1.1.1.85) is responsible for this loss of pathogenicity and thus 3-isopropylmalate dehydrogenase is a pathogenicity factor. We have demonstrated, therefore, that since 3-isopropylmalate dehydrogenase is a pathogenicity factor, it is a new target for antifungal compounds. Thus compounds designed to inhibit 3-isopropylmalate dehydrogenase, or compounds selected for their ability to inhibit *Septoria nodorum* 3-isopropylmalate dehydrogenase through biochemical screening of synthetic chemical libraries or libraries of natural substances have the potential to be commercially applicable fungicides.

We have shown that a mutant of *Septoria nodorum* which cannot synthesise leucine (a leucine auxotroph), is no longer pathogenic on its normal plant host. The ability to pathogenise this host is restored to this leucine auxotroph when exogenous leucine is included with the inoculum used to infect the host. (Example 1).

We have also shown that this leucine auxotroph is defective in the enzyme which converts 3-carboxy-2-hydroxy-4-methylpentanoate to 2-oxo-4-methyl-3-carboxy-pentanoate, more commonly know as β-isopropylmalic acid and α-ketoisocaproic acid respectively, namely 3-carboxy-2-hydroxy-4-methylpentanoate:NAD⁺ oxidoreductase (EC 1.1.1.85), more commonly know as 3-isopropylmalate dehydrogenase, (see Example 1, Example 2 and Example 3).

We have shown that we can restore to this mutant the ability to synthesise leucine and that we can restore to the mutant the ability to pathogenise its host by transforming into this mutant the gene coding for 3-isopropylmalate dehdrogenase from *Neurospora crassa* (Example 3). Furthermore we have demonstrated that we can restore leucine prototrophy and presumably pathogenicity to this leucine auxotroph by transforming into the mutant the *Septoria nodorum* wild-type allele of 3-isopropylmalate dehydrogenase (*leuA*, Example 6).

We have demonstrated that by replacing the wild-type allele (*leuA*) with a *leuA* gene disrupted by a gene coding for resistance to the antibiotic hygromycin B, we can convert wild-type *Septoria nodorum* into leucine-requiring mutant which is no longer pathogenic to its host, thus demonstrating the surprising discover that 3-isopropylmalate dehydrogenase is a pathogenicity factor of *Septoria nodorum* (Example 7).

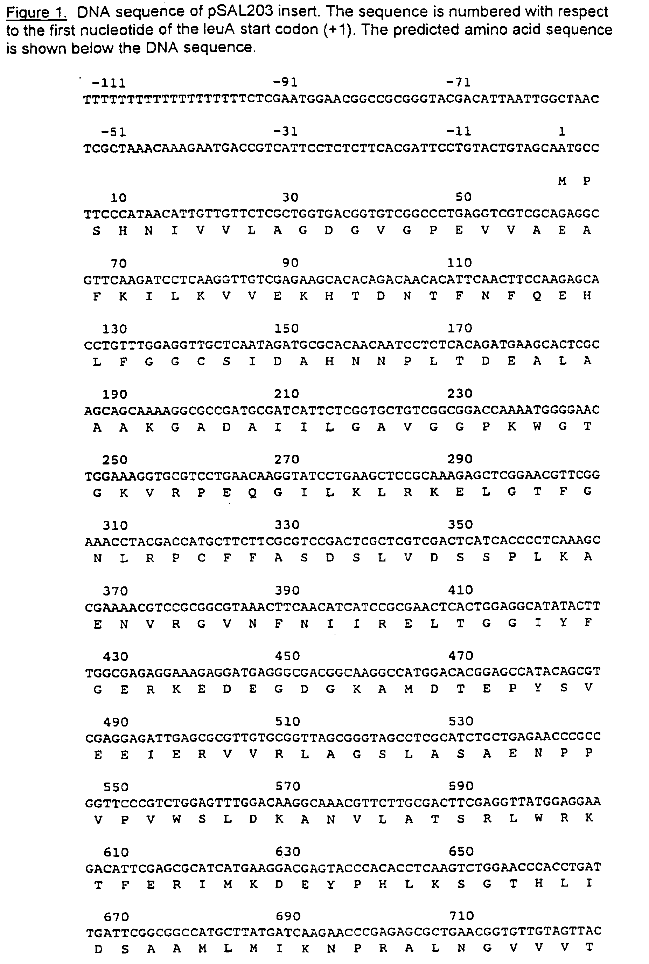

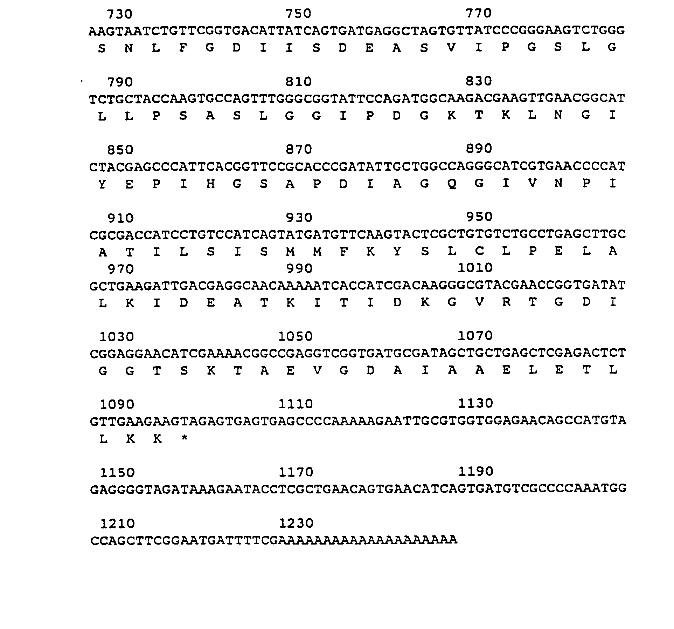

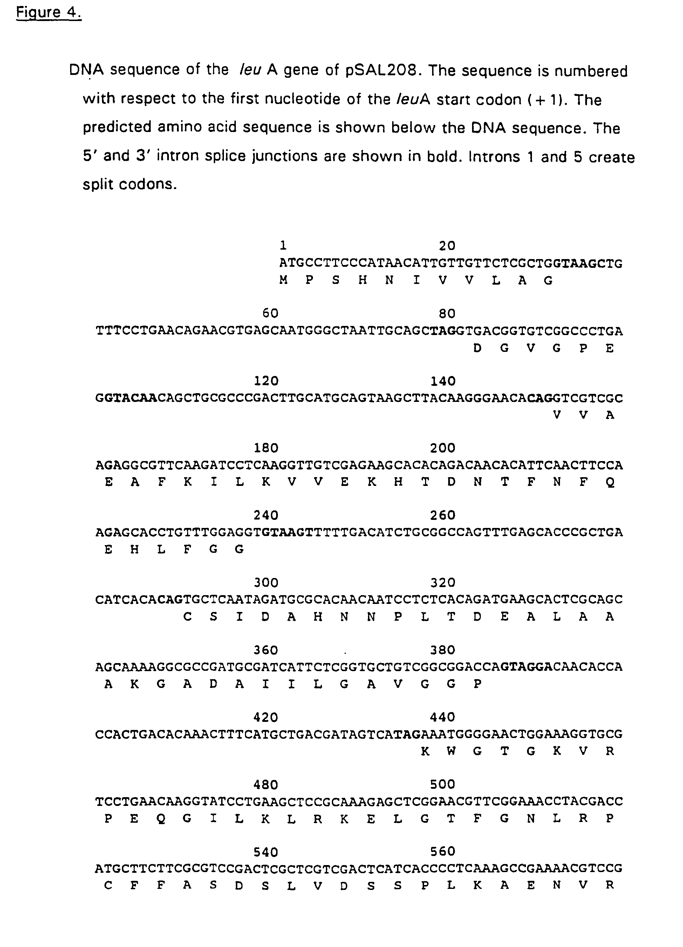

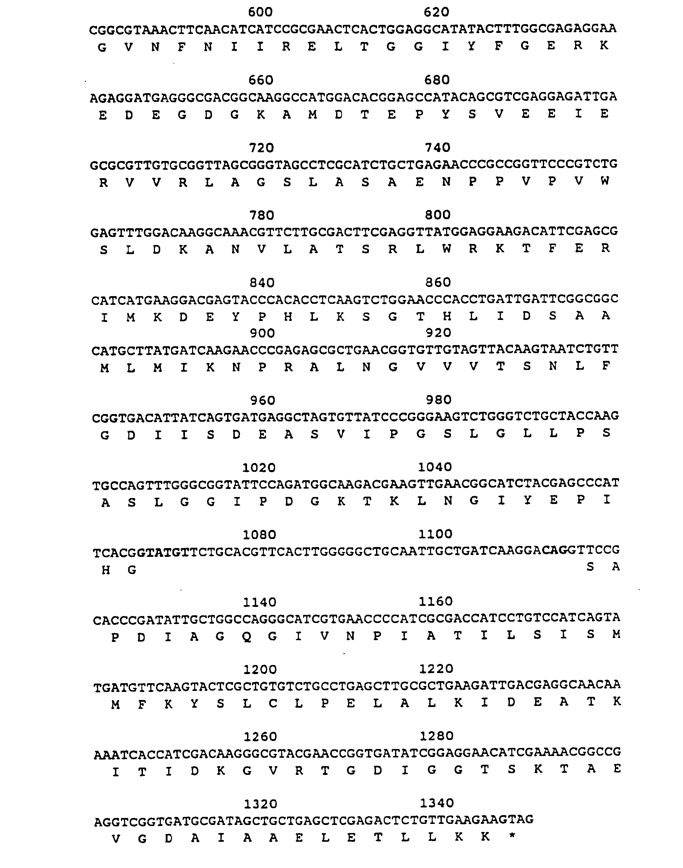

Thus, the present invention provides a method for identifying potential fungicides which comprise testing a compound in a 3-isopropylmalate dehdrogenase (IPMDH) inhibition assay where the IPMDH is the IPMDH from *Septoria nodorum* as shown in any of Figures 1, 2 and 4 and where a measurable reduction of enzyme is observed, the compound is subsequently subjected to conventional test(s) to confirm *the in vivo* fungicidal activity. The invention further provides a non-pathogenic, leucine-requiring mutant of *Septoria nodorum* which is disrupted in the 3-isopropylmalate dehydrogenase gene as shown in Figure 1 or 4.

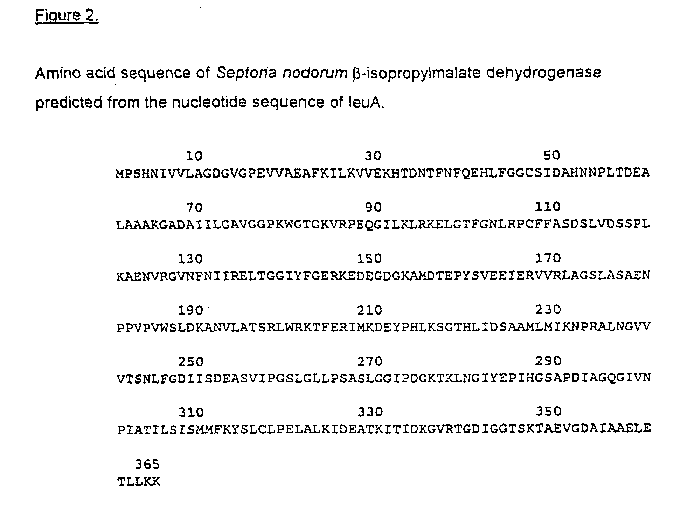

The invention further provides:
(i) the amino acid sequence as shown in Figure 2, derived from a *Septoria nodorum* gene coding for the enzyme 3-isopropylmalate dehydrogenase.
(ii) the DNA sequence from pSAL203 (NCIMB 40917) as shown in Figure 1 from a cDNA clone from *Septoria nodorum* which encodes 3-isopropylmalate dehydrogenase;
(iii) the DNA sequence of the leuA gene of *Septoria nodorum* as in Figure 4, coding for the genomic equivalent of the sequence of (ii); and
(iv) plasmid pSAL203 obtainable from plasmid pSAL203 obtainable from strain pSAL203/JA221 deposited as NCIMB 40917, plasmid pSAL204 obtainable from strain pSAL204/JA221 deposited as NCIMB 40918, plasmid pSAL205 obtainable from strain pSAL205/XL1 deposited as NCIMB 40919, plasmid pSAL206 obtainable from strain pSAL206/XL1 deposited as NCIMB 40920 or plasmid pSAL208 obtainable from strain pSAL208/XL1 deposited as NCIMB 40921.

The invention additionally provides an expression system which is:
(i) any expression system which includes an expressible form of DNA sequence from pSAL203 (NCIMB 40917) as shown in Figure 1 from a cDNA clone from *Septoria nodorum* which encodes 3-isopropylmalate dehydrogenase; or
(ii) any expression system which includes an expressible form of a 3-isopropylmalate dehydrogenase gene from *Septoria nodorum* as show in Figure 4.

The invention also provides a method of screening for a potential fungicide, the method comprising identifying a compound which inhibits *Septoria nodorum* IPMDH as shown in any of Figures 1, 2 and 4.

The invention moreover provides a method of inhibiting the growth of a fungus by applying a compound identified by a method of the invention, where the compound is selected from:
- phenyl 5-bromo-6-chloro-2-trifluoromethyl-benzimidazole-1-carboxylate;
- phenyl 4,5,6-tribromo-2-trifluoromethyl-benzimidazole-1-carboxylate; and
- 1,4-dimethylpentyl 4,5,6-tribromo-2-trifluoro methyl-benzimidazole-1-carboxylate.

The following examples demonstrate that, surprisingly, 3-isopropylmalate dehdrogenase is essential to the pathogenicity (that is it is a pathogenicity factor) of *Septoria nodorum* and establish that this enzyme is a target for the screening of fungicides. To facilitate the use of this enzyme for fungicide screening we have demonstrated the expression of *Septoria nodorum* 3-isopropylmalate dehdrogenase in *Escherichia coli* (recombinant IPMDH) and the extraction of useful levels of 3-isopropylmalate dehdrogenase activity from such clones (Example 4). Finally we demonstrate the utility of this invention by using recombinant IPMDH to discover active fungicides from a biochemical screen (examples 8 and 9)

The invention is illustrated in the following examples.

The bacterial strains referred to in the following examples were deposited with the National Collections of Industrial and Marine Bacteria (NCIMB) Ltd., 23 St Machar Drive, ABERDEEN, AB2 1RY, Scotland, UK. on the 12 February, 1998.

| NCIMB | Plasmid | Host (genotype) |
|---|---|---|
| 40917 | pSAL203 | JA221 (*hsd*R, *lac*Y, *leu*B6, *trp*E5, *rec*A1) |
| 40918 | pSAL204 | JA221 (*hsd*R, *lac*Y, *leu*B6, *trp*E5, *rec*A1) |
| 40919 | pSAL205 | XL1-BLUE (*recA1, endA1, gyrA96, thi1, hsdR17, supE44, relA1, lac[F', proAB, lacl^{q}ZΔm15, Tn10)* |
| 40920 | pSAL206 | XL1-BLUE (*recA1, endA1, gyrA96, thi1, hsdR17, supE44, relA1, lac[F', proAB, lacl^{q}ZΔm15, Tn10)* |
| 40921 | pSAL208 | XL1-BLUE (*recA1, endA1, gyrA96, thi1, hsdR17, supE44, relA1, lac[F', proAB, lacl^{q}ZΔm15, Tn10)* |
| 40922 | pSAL209 | XL1-BLUE (*recA1, endA1, gyrA96, thi1, hsdR17, supE44, relA1, lac[F', proAB, lacl^{q}ZΔm15, Tn10)* |
| 40923 | pSAL210 | XL1-BLUE *(recA1, endA1, gyrA96, thi1, hsdR17, supE44, relA1, lac[F', proAB, lacl^{q}ZΔm15, Tn10)* |
| 40924 | JA221F' | *hsd*R, *lac*Y, *leu*B6, *trp*E5, *rec*A1 {F' *pro*AB, *lac*I^{q}, *lac*ZΔM15, Tn10}. |

The *Septoria nodorum* isolates 40.1 and 40.15 have been deposited on the 11 February 1998 with CABI Bioscience, a division of CAB international, UK Centre (Egham)-International Mycological Institute, Bakeham Lane, Surrey, TW20 9TY, UK, under accession numbers IMI 378471 and IMI 378472, respectively.

### Example 1

### Demonstration that a Leucine Auxotrophic Mutant of Septoria nodorum is Non-pathogenic

Conidia of *Septoria nodorum* (Berks; *Leptosphaeria nodorum,* Muller, strain 40-1) were irradiated with UV light at a dose of 1296-1440J. At these doses of UV irradiation, normally 1-5% of the treated conidia survived. In total 6 000 survivors of the UV irradiation were tested for their ability to grow on a complex medium (PDA: Potato Dextrose Agar, from Oxoid supplied by Unipath Limited., Basingstoke, Hampshire, England. The medium was made to the manufacturer's instructions) compared to a defined, minimal medium (Cz: Czapek Dox Agar [Modified], from Oxoid supplied by Unipath Limited., Basingstoke, Hampshire, England. The medium was made to the manufacturer's instructions). Of the 6 000 survivors of the UV irradiation, thirty-one were able to grow on the complex medium but were unable to grow on the defined minimal medium. Mutants that exhibit this characteristic are auxotrophic mutants.

Those familiar with nutritional analysis of auxotrophic mutants will be aware that Holliday solutions (Holliday, R., 1956, Nature 178, 987) can be used with a defined minimal medium to characterise precisely the nutritional requirements of any particular auxotrophic mutant. Of the thirty-one auxotrophic mutants tested, one mutant (hereinafter called 40-15) required leucine (added at 40µg/ml to Cz medium) to grow on the defined minimal medium, implying that 40-15 was incapable of synthesising leucine and was, therefore a leucine auxotroph. Those skilled in the art of analysing auxotrophic mutants (auxanography) will know that feeding the precursors of leucine to a leucine auxotroph can help to elucidate which particular step is defective in that biosynthetic pathway. Thus by adding, in separate growth tests some of the known precursors of leucine biosynthesis: pyruvate; α-ketoisovalerate; α-isopropylmalate; β-isopropylmalate and α-ketoisocaproate (all supplied by Sigma Chemical, Sigma-Aldrich Company Ltd., Fancy Road Pool, Dorset, UK except for β-isopropylmalate which was supplied by Wako Chemicals GmbH, Nissanstraβe, D-41468 Neuss, Germany) at 40µg/ml in Cz plates the biochemical lesion in the leucine-requiring auxotroph 40-15 could be inferred. This auxanographic analysis strongly implicated that 3-isopropylmalate dehydrogenase was defective in 40-15 since only leucine and α-ketoisocaproate restored normal growth to 40-15 whereas β-isopropylmalate, α-ketoisovalerate and pyruvate did not. These conclusions were confirmed by biochemical analysis (Example 2) and genetical analysis (Example 3).

To assess the effect that leucine auxotrophy had on the pathogenicity of *Septoria nodorum* 40-15, the mutant was tested first in a detached leaf assay and then on whole plants.

The detached leaf assay was that described by A C Newton and C E Caten (1988),Trans. Br. Mycol. Soc. 90, 199. The tips (2 cm) of 10 day old wheat (Fenman) plants were cut and placed on tap water agar (0.5g of Oxoid agar No. 3 [from Oxoid supplied by Unipath Limited., Basingstoke, Hampshire, England] in 85 ml of water, which was autoclaved and to which was added benzimidazole [supplied by Sigma Chemical UK.] at a final concentration of 150µg/litre, dispensed into 5 cm Petri dishes. To these detached leaves were inoculated 5µl of a 10⁶ conidia/ml conidial suspension in 0.02% Tween 20 (supplied by Sigma Chemical UK). The inoculated plants were incubated at 18 °C with continuous illumination by near ultraviolet and white light and the pathogenicity of 40-15 compared to a wild-type *Septoria nodorum* (40-1) was assessed after one week. Pathogenicity was scored on a scale of zero to five for increasing chlorosis and necrosis, with a score of zero indicating that a leaf segment remained completely uninfected and a score of five indicating that a lesion comparable in size to the chlorotic and necrotic symptoms of the wild-type infection had formed during the period of the assessment.

In whole plant assays, the wheat cultivar Fenman at the two leaf stage was inoculated with about 5x10⁵ conidia and assessed for the degree of damage caused by 40-15 compared with a wild-type isolate after 14 days incubation at high humidity 20 °C.

In the detached leaf assays, the leucine auxotroph would typically give a pathogenicity score of zero and on whole plants the number of lesions would be about 10% of the wild-type. Furthermore, no *Septoria nodorum* 40-15 could be reisolated from these lesions, implying that the lesions that were seen on plants inoculated with 40-15 were not due the growth of 40-15. The pathogenic response of 40-15 could be enhanced by including leucine (at 40µg/ml) in the 40-15 conidial inoculum in both the detached leaf and the whole plant assays.

These observations demonstrate that leucine metabolism is essential for the pathogenicity of *Septoria nodorum*. The data also imply that, in the case of the mutant 40-15, it is the loss of the enzyme activity 3-isopropylmalate dehydrogenase that has caused the loss of leucine metabolism. The following examples will demonstrate that this is the case.

### Example 2

### 3-Isopropylmalate Dehyrogenase Activity of Wild-Type Septoria nodorum (40-1) Compared with that of the Leucine Requiring Mutant 40-15

Typically *Septoria nodorum* cultures were grown in liquid Cz medium with leucine included at 40 µg ml⁻¹. *Septoria* spores were added at 5.0 x10⁵ spores for each ml of 200 ml of growth medium in 1 litre flasks. After 3 days at 25 °C, with constant shaking at 220 rpm, mycelia of the wild-type (40-1) and 40-15 were harvested by centrifugation for 40 minutes at 8000 g and 4°C. The mycelia were ruptured by grinding for three minutes in an ice cold pestle and mortar with an equal volume of sand and an equal volume of ice cold extraction buffer (100 mM KH₂PO₄, 100 mM KOH, pH 7.0, with 0.5 mg/ml bovine serum albumin, 3.75 mM dithiothreitol and 50 µM manganese chloride). The mycelial extracts were clarified by centrifugation for ten minutes at 13,000 g and 4°C, and desalted using a Pharmacia PD-10 column (Pharmacia Biotech, 23 Grosvenor Road, St. Albans, Herts. AL1 3AW, UK.) pre-equilibrated with assay buffer (100 mM KH₂PO₄, 100 mM KOH, pH 8.0, containing 0.5 mg/ml BSA, 3.75 mM dithiothreitol and 50 µM manganese chloride).

The clarified and desalted extracts of 40.1 and 40.15 were assayed for 3-isopropylmalate dehydrogenase activity by monitoring the conversion of the substrate, 3-isopropylmalate (3-IPM) to the product, α-ketoisocaproate (α-KIC) using a HPLC to resolve and quantify the product and substrate. In a final volume of 1 ml, 820 µl of extract was incubated at 30°C with 50 mM KCI, 0.5 mM manganese chloride, 1 mM nicotinamide adenine dinucleotide (NAD⁺) and 2 mM D,L-threo-3-isopropylmalic acid (3-IPM). After 60 minutes the enzyme reaction was stopped by adding 200µL of 2M perchloric acid, followed by neutralisation with 100µL of 1 M potassium hydroxide to precipitate the protein present in the assay.

Precipitated protein was removed by centrifugation at 13,000 rpm for five minutes. The resultant clarified reaction mixture was subjected to isocratic HPLC analysis using an Aminex HPX-87H column (300 x 7.8 mM; Bio-Rad® Laboratories) with 0.005M H₂SO₄ as mobile phase, at a flow rate of 0.4 ml/min. The column eluate was monitored at 210 nM. Substrate and product peak areas were converted to millimolar concentration values using calibration curves in the range of 0.01-2.00 mM for both the substrate and the product. The conversion (in percent) of the substrate to the product was calculated using the following expression:
%conversion=100x [(α-KIC at 60 minutes)-(α-KIC at 0 minutes)/(3-IPM concentration at 0 minutes)].
The assays were done twice and the controls were done by stopping the reaction at time zero minutes. NAD⁺ was omitted from the incubation to demonstrate that the conversion of 3-IPM to α-ketoisocaproate was dependent on the presence of the coenzyme, NAD⁺.

The results are presented in the table below:

| | **Conversion of 3-isopropylmalate to α-ketoisocaproate (%)** | |
|---|---|---|
| | + NAD⁺ | - NAD⁺ |
| 40-1 | 8 | 0 |
| 40-15 | 0 | 0 |

The data for both the wild-type *Septoria nodorum* (40-1) demonstrate that coenzyme-dependent 3-isopropylmalate dehydrogenase activity was detected in the wild-type 40.1 but not in the leucine auxotrophic mutant, 40-15 and thus provides biochemical evidence that 3-isopropylmalate dehydrogenase activity is lacking in mutant 40-15. Example 3 provides the genetical evidence that it is the 3-isopropylmalate dehydrogenase gene that is defective in 40-15.

### Example 3

### Genetic Complementation of the Leucine Mutant 40-15 with a Gene Coding for 3-Isopropylmalate Dehydrogenase from Neurospora crassa

Those skilled in the art of genetic transformation are aware that when a gene representing an enzyme deficient in an auxotrophic mutant (the wild-type allele) is transferred into that mutant, it so functions, *in vivo,* that the biochemical lesion is compensated for by the newly added gene. This process is called genetic complementation. The complementing gene does not need to originate from an organism of the same species as the auxotrophic mutant. Thus, in this example, the defect in the leucine auxotrophic mutant was repaired by the addition of a 3-isopropylmalate dehydrogenase gene from the non-pathogenic fungus *Neurospora crassa*.

The *Septoria nodorum* leucine mutant 40-15 could be restored to prototrophy by complementation with the 3-isopropylmalate dehydrogenase gene (*leu*-1) from *Neurospora crassa*. The *leu*-1 gene was introduced into *Septoria nodorum* 40-15 on the plasmid vector pLEU12 (kindly provided by Jarai *et al*., 1990, Mol. Gen. Genet. **224,** 383), and, at the same time, with the plasmid pAN7-1 (kindly provided by Punt *et al*, 1987, Gene **56,** 117), a vector which confers resistance to the antifungal compound hygromycin B. This process of co-transformation is well known to those competent in genetic transformation of fungi (Cooley *et al*. (1990) Mycol. Res. 94, 145). Colonies which were resistant to 200 µg/ml hygromycin B (Sigma Chemical) were selected for by growth on Cz agar including hygromycin B and 1 M sucrose (Sigma Chemical) supplemented with leucine at 40µg/ml. Hygromycin-resistant colonies from this selection were inoculated to Cz medium without leucine. In a separate treatment, hygromycin resistant colonies of 40-15 were also generated by using pAN7-1 alone. All hygromycin-resistant colonies originating from co-transformation conditions (thirty-two of thirty-two tested) were able to grow on media lacking leucine, whereas none of the hygromycin-resistant colonies from the pAN7-1 alone transformation conditions (of 13 tested) were able to grow.

Four co-transformants and four pAN7-1 transformants were tested for the acquisition of the *Neurospora crassa leu*-1 gene by probing their genomic DNA in Southern hybridisations by the method described by T Maniatis, E F Fritsch and J Sambrook (1982) in "Molecular Cloning, A Laboratory Manual" published by the Cold Spring Harbor Laboratory, using a P³² labelled *leu*-1-specific PCR product amplified from pLEU12. All four leucine prototrophic co-transformants produced positive signals consistent with complex multiple integration events of pLEU12, whereas no signals were produced for the pAN7-1 transformants. These data unequivocally demonstrate that when the leucine auxotrophic mutant of *Septoria nodorum* (40-15) acquires a 3-isopropylmalate dehydrogenase gene, prototrophy is restored. These data demonstrate therefore that, in this mutant, it is the gene for 3-isopropylmalate dehydrogenase which is deficient or perturbed.

To test what influence restoration to prototrophy had on the pathogenicity of co-transformants, detached wheat leaf assays, as described previously, were done, using the variety Fenman, using six leucine prototrophic co-transformants and four leucine auxotrophic pAN7-1 transformants. Inoculations were made by transferring macerated mycelium, which had been grown on either Cz medium (for the co-transformants) or Cz medium supplemented with leucine (for the pAN7-1 transformants). None of the pAN7-1 transformants showed any sign of growth, whilst the co-transformants were able to grow across the leaf surface. The co-transformants also produced some necrosis on prolonged incubation (10 days). The lesions produced by the co-transformants were somewhat atypical, probably due to the method of inoculation and failed to demonstrate the same level of pathogenicity as the original wild-type strain. The pAN7-1 transformants produced neither growth nor lesions, demonstrating that the gene for 3-isopropylmalate dehydrogenase is required for pathogenicity. The results of the assessment, done in triplicate in two independent experiments, are summarised in the table below.

| | EXPERIMENT 1 | | | EXPERIMENT 2 | | |
|---|---|---|---|---|---|---|
| | TOP OF LEAF | | BOTTOM | TOP OF LEAF | | BOTTOM |
| | GROWTH | LESION | LESION | GROWTH | LESION | LESION |
| 40-15 | 0 | 0 | 0 | 0 | 0 | 0 |
| WILD-TYPE | 3 | 4 | 4 | 2 | 4 | 5 |
| 40-15 /pAN7-1^{*} | 0 | 1 | 1 | 0 | 1 | 1 |
| 40-15 /pAN7-1 /pLEU12^{†} | 3 | 2 | 2 | 2 | 2 | 2 |

| | | | | | | |
|---|---|---|---|---|---|---|
| * The leucine auxotroph (40-15) transformed with the plasmid pAN7-1. † The leucine auxotroph (40-15) co-transformed with the vector pAN7-1 and the *Neurospora crassa* 3-isopropylmalate dehydrogenase gene (*leu*-1) on plasmid pLEU12. | | | | | | |

The data presented in Example 3 show that a functional 3-isopropylmalate dehydrogenase gene is essential for the pathogenicity of *Septoria nodorum*.

### Example 4

### Cloning and Expression of the 3-isopropylmalate dehydrogenase Gene from Septoria nodorum

A clone of the *S nodorum* 3-isopropylmalate dehydrogenase gene (hereinafter called *leu*A) was isolated by complementation of an *E coli leu*B mutant (itself deficient for the enzyme 3-isopropylmalate dehydrogenase) using an *S nodorum* cDNA library constructed from mRNA isolated from a wild-type.

*S nodorum* mycelium was produced from BS171, a wild-type isolate of the pathogen (Osbourn, A. E., Scott, P. R. and Caten, C. E., 1986, Plant Pathol. 35, 135-145) grown in liquid Cz medium. Mycelium was harvested, frozen in liquid nitrogen and RNA isolated using a RNA Isolation Kit (Stratagene, Cambridge Innovation Centre, Cambridge Science Park, Milton Road, Cambridge CB4 4GF, UK) in accordance with manufacturer's recommendations. Messenger RNA (mRNA) was purified from total RNA using an oligoTex mRNA Mini Kit (Qiagen Ltd., Unit 1, Tillingbourne Court, Dorking Business Park, Station Road, Dorking, Surrey RH10 1HJ, UK ) and was then used to construct a cDNA library with a ZAP-cDNA Synthesis Kit from Stratagene. The primary titre of this library was approximately 10⁶ pfu ml⁻¹, with a recombinant frequency of 99.2 % and an insert size range of 0.25-2.5 kb (average 0.9 kb). The primary library was amplified to give a titre of 3 × 10⁹ pfu ml⁻¹ and then mass excised using Exassist helper phage and strain SOLR in accordance with manufacturer's recommendations (Stratagene) to produce a phagemid library with a titre of 5 × 10⁸ cfu ml⁻¹.

A *leu*B6 recipient *E coli* strain was made by introducing the F episome from *E coli* XL1 BLUE (Stratagene) into *E coli* JA221 (ATCC 33875 Carbon, J., 1978, J. Mol. Biol. 120, 517-532) to produce JA221 F' (*hsd*R, *lac*Y, *leu*B6, *trp*E5, *rec*A1 {F' *pro*AB, *lac*I^{q}, *lac*ZΔM15, Tn10}). JA221F' cells were transfected with the phagemid library, rinsed with 10 mM MgSO₄.7H₂O and plated on GB1 minimal media (Oxoid Bacteriological agar No.1 at 15g, glucose at 0.4% w/v, thiamine at 2.5 mg, MgSO₄.7H₂O at 1 mM, KH₂PO₄ at 3.4g, (NH₄)₂SO₄ at 0.5g and a trace of FeSO₄.7H₂O a litre) containing 100 µg ml⁻¹ ampicillin, 15 µg ml⁻¹ tetracycline, 50µM isopropyl-β-thiogalactopyranoside (IPTG) and 40 µg ml⁻¹ tryptophan. After one day of growth at 37°C leucine prototrophic colonies were seen at a frequency of approximately 1 for every 7 × 10⁵ transfectants. Plasmid DNA from a selection of these was isolated by methods described in Maniatis *et al* and transformed into JA221 where equivalent transformation frequencies for ampicillin resistance and leucine prototrophy were observed implying that the cDNA for 3-isopropylmalate dehydrogenase had been isolated from the cDNA library.

The DNA sequence of the cDNA insert of one of the complementing clones (pSAL203) was determined using the DNA sequencing facilities of Alta Biosciences, (School of Biochemistry, University of Birmingham, Edgbaston, Birmingham. B15 2TT, UK) and the open reading frame of *leu*A identified by comparison with the predicted amino acid sequence of the *N crassa leu*1 gene (Figure 1). The *leu*A gene encodes a 365 amino acid polypeptide (Figure 2), three amino acids shorter than the *leu*1 polypeptide from *N crassa* with which it shares 68.8% identity.

Overexpression of *leu*A was achieved by sub-cloning the open reading frame (ORF) of *leu*A into the vector pJLA503 (Schauder et al, Gene (1972), 52, 279) to make pSAL204. The *leu*A gene was amplified by PCR using pSAL203 as a template at 10 ng of DNA in a 50 µl reaction with buffer and *Taq*1 polymerase supplied by Boehringer Mannheim (Boehringer Mannheim [Diagnostics and Biochemicals] Ltd., Bell Lane, Lewes, East Sussex, BN7 1 LG, UK) and in conditions recommended by the suppliers. The primers were used at 50 pmoles a reaction using a primer with an *Ndel* restriction site (in bold and underlined) at the start of the *leu*A ORF 5'-GCATGCA**CATATG**CCTTCCCATAAC-3' and an *EcoR*I restriction site (in bold and underlined) downstream from the stop codon of the ORF, GCAT**GAATTC**TGTTCACTGTTCAGC. The reactants were incubated for one cycle at 93°C, 4 minutes; 47°C, 1 minute; 72°C, 1 minute 30 seconds; then 10 cycles of 93°C, 1 minute; 47°C, 1 minute; 72°C, 1 minute 30 seconds; then 10 cycles of 93°C, 1 minute; 47°C, 1 minute; 72°C, 2 minutes; then 10 cycles of 93°C, 1 minute; 47°C, 1 minute; 72°C 2 minutes 30 seconds; then, finally 8 minutes at 72°C. The amplified product was cloned into pJLA503 as an *Nde*I-*Eco*RI fragment using the restriction sites on the primers.

Using the growth conditions described by Schauder *et al*, over-expression of the *leu*A gene was demonstrated by harvesting cells from 100 ml of liquid medium by centrifugation for 10 minutes at 8,000 g, 4°C, rupturing the cell pellet in 4 ml of assay buffer (100 mM KH₂PO₄ and 100 mM KOH at pH 8.0, also containing 50 mM KCI) followed by desalting using a Pharmacia PD-10 column pre-equilibrated with assay buffer.

The assay for 3-isopropylmalate dehydrogenase activity was essentially that described by Parsons, S J and Burns, R O (1970) in Methods Enzymol. 17A, 793-799. Clarified desalted extracts were assayed for 3-isopropylmalate dehydrogenase activity by monitoring the appearance of the reduced form of the coenzyme, spectrophotometrically at 340 nm. In a final volume of 1 ml, 10-860 µl of extract was incubated at 30°C with 0.5 mM manganese chloride, 1 mM nicotinamide adenine dinucleotide (NAD⁺) and 2 mM D,L-threo-3-isopropylmalic acid (3-IPM). The reaction was started by the addition of the substrate (3-IPM), and the change in absorbance at 340 nm was recorded by a spectrophotometer for two minutes thereafter. Assays were carried out in duplicate, and omission of the substrate (3-IPM) from the incubation mixture was used to demonstrate that increase in absorbance at 340 nm was substrate-dependent. The rate of NADH production in a given unit of time was taken from the initial linear portion of the reaction progress curve. The protein concentration of extracts was determined using the method described by Bradford, M. (1976) Anal. Bioch., 72, 248-254.

To facilitate comparisons between different clones, 3-isopropylmalate dehydrogenase activities were expressed as the quantity of NADH produced in a unit of time for the quantity of protein used in the assay, and was calculated using the following expression where the 3-isopropylmalate dehydrogenase activity (µmole/min/mg) = [(rate (+IPM) - rate (-IPM)]/ 6.22 x protein concentration, and the value 6.22 is the absorbance at 340 nm of 1 µ mole/ml solution of NADH.

The data are given in the table below, and show that pSAL204 contains a functional 3-isopropylmalate dehydrogenase gene because the *leu*B mutant of *E coli* has demonstrable 3-isopropylmalate dehydrogenase activity only when pSAL204 is present. Cloned into pJLA503, the 3-isopropylmalate dehydrogenase gene has elevated levels of 3-isopropylmalate dehydrogenase activity.

| **HOST** | **VECTOR** | **IPMDH ACTIVITY (nmol/min/mg)** |
|---|---|---|
| **XL1-BLUE** *recA1, endA1, gyrA96, thi1, hsdR17, supE44, relA1, lac[F', proAB, lacI^{q}ZΔm15, Tn10]* | pSAL204 | 547 |
| **XL1-BLUE** *recA1, endA1, gyrA96, thi1, hsdR17, supE44, relA1, lac[F', proAB, lacI^{q}ZΔm15, Tn10]* | pJLA503 | 27 |
| **JA221** *hsdR, lacY, leuB6, trpE5, recA* | pSAL204 | 218 |
| **JA221** *hsdR, lacY,* /*euB6, trpE5, recA* | pJLA503 | no activity |

These data clearly demonstrate the utility of the invention in that pSAL204 is now expressing a pathogenicity factor of *Septoria nodorum,* 3-isopropylmalate dehydrogenase, in the bacterium *Escherichia coli*. In this genetic background the enzyme is easily isolated and a facile biochemical assay is possible.

### Example 5

### Cloning the Genomic Form of 3-Isopropylmalate Dehydrogenase Gene from Septoria nodorum

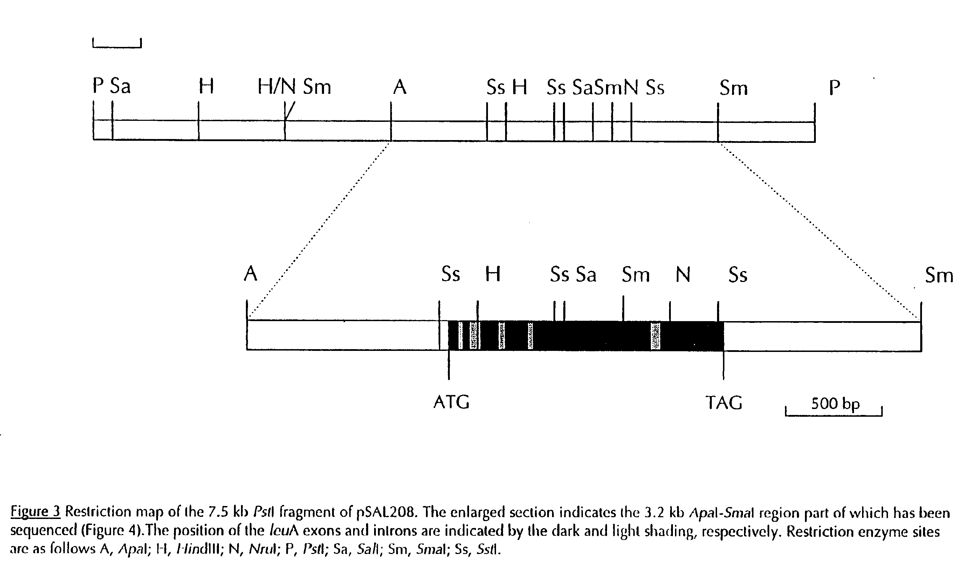

A clone of the genomic form of *leu*A was isolated from a *Septoria nodorum* cosmid library using the cDNA clone as a probe. The cosmid library was constructed from a benomyl-resistant mutant (BSm300) of strain BS171 and has been previously described by Cooley et al, (J Gen Microbiol, 1991, 137, 2085-2091). The screening of the cosmid library was that described by Maniatis *et al*, where approximately 3 × 10³ colonies were plated on to Duralose (Stratagene) membranes, replicated and probed with a fragment of pSAL203 which represented the 3-isopropylmalate dehydrogenase gene. Two duplicate signals were produced and their corresponding colonies purified and rescreened by probing. Cosmid DNA (pSAL205 and pSAL206 ), was isolated from these colonies, digested with a selection of restriction enzymes and probed in a Southern hybridisation with the same pSAL203 fragment used to isolate the cosmids. Each cosmid produced signals corresponding to a 7.5 kb fragment when digested with *Pst*I. A similar signal was produced when Pstl-digested BSm300 genomic DNA was probed in Southern hybridisations with the same insert, thereby demonstrating the integrity of the cosmid insert. The 7.5 kb *Pst*I fragment of pSAL206 was subcloned into pBLUESCRIPT(SK⁺) to produce pSAL208 and this plasmid restriction mapped (Figure 3).

The DNA sequence of the open reading frame of 3-isopropylmalate dehydrogenase in pSAL208 has been determined (Figure 4) and comparison with the cDNA sequence has revealed the presence of five introns. These introns are short, approximately 50 bp in length, and all possess consensus 5' and 3' splice sites (Gurr, S. J., Unkles, S.E. and Kinghorn, J. R., 1987 in Gene Structure in Eukaryotic Microbes, Chapter 5, 93-139, Kinghorn, J. R. (ed), IRL Press, Oxford).

### Example 6

### The Genomic Form of 3-Isopropylmalate Dehydrogenase Restores Prototropy to Septoria nodorum (40.15)

The genomic form of the S *nodorum leu*A gene was transformed into the leucine auxotroph 40.15 by co-transformation, where it restored prototrophy. Protoplasts of 40.15 were treated with a mixture of pAN7-1 and pSAL208 DNA and hygromycin B resistant colonies were selected initially in the presence of leucine. Thirty-one out of thirty-two of these hygromycin B resistant transformants were able to grow on media lacking leucine, whereas as seen in earlier examples, hygromycin B resistant transformants treated with pAN7-1 alone would not grow on minimal medium unless leucine was added. These observations clearly demonstrate that the gene encoded by pSAL208 is the genomic form of 3-isopropylmalate dehydrogenase.

### Example 7

### Disruption of the Genomic Form of 3-isopropylmalate dehydrogenase in Septoria nodorum, and the Effect that this has on Pathogenicity of Septoria nodorum

The *leu*A gene of wild type strain BS171 has been mutated by gene disruption using a gene replacement approach. The disruption construct was produced firstly by excising a 3.1 kb Apal fragment from pSAL208 to give pSAL209. The 1.0kb *Hin*dIII-*Nru*I fragment of pSAL209 internal to *leu*A was then replaced with the 3.65kb *Hin*dIII*-Stu*I fragment of pAN7-1 carrying the hygromycin B resistance cassette, a strategy well known to those skilled in the art. The resulting vector (pSAL210) was linearised by digestion with *Apa*I and transformed into BS171 by selecting for hygromycin B resistance in the presence of leucine. A total of eight hygromycin B resistant colonies were produced and these were inoculated to minimal medium lacking leucine. Four out of the eight hygromycin B resistant transformants failed to grow in the absence of leucine suggesting that these had been disrupted at the *leu*A locus.

Genomic DNA from all eight transformants was isolated, digested with *Pst*I and probed in Southern hybridisations with a *leu*A-specific probe. DNA from a wild type strain and from the four leucine prototrophic, hygromycin B resistant transformants described above produced a signal corresponding to a 7.5 kb fragment indicating that the *leu*A gene was intact. Signals of varying size were also seen in the prototrophic, hygromycin B resistant transformants which are consistent with ectopic integration of the transforming DNA. The four leucine auxotrophic, hygromycin B resistant transformants were missing the 7.5kb fragment. The only signal seen in these transformants was a 4.3 kb fragment, consistent with the doublet expected to be produced in the case of a homologous gene replacement at the *leu*A locus. These data indicate that the leucine auxotrophs produced by gene disruption are disrupted only at the *leu*A locus.

The pathogenicity of the eight hygromycin B resistant transformants produced in this example were tested in detached leaf assays as described in the earlier examples. Detached Fenman leaves were inoculated with 5 µl of a conidial suspension at a concentration of 5 × 10⁵ conidia ml⁻¹ in 0.01% Tween 20 in triplicate. After incubation the following results were observed.

| STRAIN | PATHOGENICITY SCORE |
|---|---|
| CONTROL | 0 |
| WILD-TYPE | 5 |
| LEU⁺ TRANSFORMANT* | 5 |
| LEU⁻ DISRUPTANT§ | 0 |

| | |
|---|---|
| * Leucine prototrophic transformant with ectopic integration of pSAL210 § Leucine auxotrophic transformant with disruption of *leu*A | |

All of the leucine prototrophic transformants which had undergone ectopic integration events were still pathogenic on wheat, whilst the leucine auxotrophic *leu*A disruptants were non-pathogenic. This clearly demonstrates the requirement of a functional 3-isopropylmalate dehydrogenase for pathogenicity in *Septoria nodorum.*

### Example 8

### Using Recombinant IPMDH in a Biochemical Screen to Discover Inhibitors of 3-isopropylmalate dehydrogenase

The recombinant IPMDH was used in a biochemical screen for inhibitors of 3-isopropylmalate dehydrogenase as follows. The assay was originally described by Parsons in 1969 (Purification and properties of β-IPMD, Parsons et al., Journal of Biological Chemistry, 244, (4), 996-1003. It is an spectrometric assay, based on the reduction of coenzyme β-NADH measured at 340 nm. Prior to any assay the rate of production of a given unit of β-NADH was determined and only the rate of the initial linear fraction of the progress curve was taken for the evaluated assay. The reagents for the assay are: the enzyme 3-Isopropyl malate dehydrogenase, KH₂PO₄, MnCl₂, β-NAD (nicotinamide adenine dinucleotide) and the substrate isopropylmalate. A typical procedure of the automated assay is: microtiter plates are prepared containing 10µl of the potential inhibitors, an enzyme solution of 10µl (enzyme is diluted 1/100 in buffer) is dispensed. The mixture is incubated for two minutes at 25°C and the reaction is started by adding 140µl buffer containing the substrate IPM, KH₂PO₄, MnCl₂, β-NAD at pH8.

The rate of the increase β-NADH is monitored each minute and five data points are collected. The inhibition of the potential inhibitors are expressed in %-inhibition using buffer as a control. Compounds which are considered to decrease enzyme activity significantly are re-tested to avoid false positive results.

In a demonstration of the utility of the invention, approximately 8 000 chemical compounds were screened for their ability to inhibit the recombinant IPMDH in assay conditions described above. Tabulated below are a selection of compounds which gave a significant inhibition of recombinant IPMDH. These compounds were also able to inhibit *Septoria nodorum in vitro*. The *in vitro* inhibition of *Septoria nodorum* (BS171) was determined by assessing the potency of each compound at 10,25, 50, 75, 100, 150, 200, and 250 parts per million (ppm) in Cz medium prepared as described in example 1. Approximately 10⁵ conidia of *Septoria nodorum* BS171 were inoculated to Cz medium with the compounds and the effect on fungal growth assessed after six days.

| **Compound** | **% inhibition at 3.3 ppm** | **Repeat** | **MIC of *Septoria nodorum in vitro* (ppm)** |
|---|---|---|---|
| phenyl 5-bromo-6-chloro-2-trifluoromethyl-benzimidazole-1-carboxylate | 33 | 56 | 100-150 |
| phenyl 4,5,6-tribromo-2-trifluoromethyl-benzimidazole-1-carboxylate | 33 | 42 | >250 |
| 1,4-dimethylpentyl 4,5,6-tribromo-2-trifluoromethyl-benzimidazole-1-carboxylate | 57 | 20 | 100-150 |
| 5,10-dioxo-5,10-dihydronaphtho-[2,3-b][1,4]dithiine-2,3-dicarbonitrile | 41 | 66 | 0-10 |

### Example 9

This examples illustrates that compounds which have shown in a biochemical screen to be inhibitors of 3-isopropylmalate dehydrogenase have *in vivo* fungicidal activity.

The compounds tested in Example 8 are assessed for activity against one or more of the following:
*Phytophthora infestans;* late tomato or potato blight
*Plasmopara viticola*: vine downy mildew
*Erysiphe cichoracearum*: cucumber powdery mildew

Aqueous solutions or dispersions of the compounds at a desired concentration, including a wetting agent, were applied by spray or by drenching the stem base of the test plants, as appropriate. Plants or plant parts were then inoculated with appropriate test pathogens and kept under controlled environment conditions suitable for maintaining plant growth and development of the disease. After an appropriate time, the degree of infection of the affected part of the plant was visually estimated. Compounds are assessed on a score of 1 to 8 where the scores have the following meanings:

| Score | % control |
|---|---|
| 0 | 0-20 |
| 1 | 20-34 |
| 2 | 35-44 |
| 3 | 45-54 |
| 4 | 55-64 |
| 5 | 65-74 |
| 6 | 75-84 |
| 7 | 85-94 |
| 8 | 95-100 |

The result are as follows. Scores are at 500 ppm unless stated

| | Score | | | |
|---|---|---|---|---|
| | Compound 1 | Compound 2 | Compound 3 | Compound 4 |
| *Phytophthora infestans* | 7 | 6 | NT | 7 |
| *Plasmopara viticola* | 5 (@125 ppm) | 6 (@125 ppm) | 8 | 7 (@125 ppm) |
| *Erysiphe cichoracearum* | 6 | 7 | 8 | 0 |

## Claims

1. A method for identifying potential fungicides which comprises testing a compound in a 3-isopropylmalate dehydrogenase (IPMDH) inhibition assay where the IPMDH is the IPMDH from *Septoria nodorum* as exemplified in any of Figures 1, 2 and 4 and where a measurable reduction of enzyme is observed, the compound is subsequently subjected to conventional test(s) to confirm the *in vivo* fungicidal activity.

2. The method according to claim 1 in which a vector is used to produce the 3-isopropylmalate dehydrogenase enzyme, wherein the vector is:
(i) any expression system which includes an expressible form of DNA sequence from pSAL203 (NCIMB 40917) as exemplified in Figure 1 from a cDNA clone from *Septoria nodorum* which encodes 3-isopropylmalate dehydrogenase; or
(ii) any expression system which includes an expressible form of 3-isopropylmalate dehydrogenase gene from *Septoria nodorum* as shown in Figure 4.

3. Non-pathogenic, leucine-requiring mutant of *Septoria nodorum* which is disrupted in the 3-isopropylmalate dehydrogenase gene as exemplified in Figure 1 or 4.

4. A non-pathogenic leucine-requiring *Septoria nodorum* mutant according to claim 3, known as 40.15 deposited under the accession number IMI 378472.

5. The amino acid sequence as exemplified in Figure 2, derived from a *Septoria nodorum* gene coding for the enzyme 3-isopropylmalate dehydrogenase.

6. The DNA sequence from pSAL203 (NCIMB 40917) as exemplified in Figure 1 from a cDNA clone from *Septoria nodorum* which encodes 3-isopropylmalate dehydrogenase.

7. DNA sequences with enough similarity to the sequence in claim 6 to encode a polypeptide with the same amino acid sequence claimed in claim 5 and derived from a plant pathogenic fungus.

8. The DNA sequence of the leuA gene of *Septoria nodorum* as exemplified in Figure 4, coding for the genomic equivalent of the sequence in claim 7.

9. Plasmid pSAL203 obtainable from strain pSAL203/JA221 deposited as NCIMB 40917, plasmid pSAL204 obtainable from strain pSAL204/JA221 deposited as NCIMB 40918, plasmid pSAL205 obtainable from strain pSAL205/XL1 deposited as NCIMB 40919, plasmid pSAL206 obtainable from strain pSAL206/XL1 deposited as NCIMB 40920 or plasmid pSAL208 obtainable from strain pSAL208/XL1 deposited as NCIMB 40921.

10. An expression system as defined in claim 2.

11. A method of screening for a potential fungicide, the method comprising identifying a compound which inhibits *Septoria nodorum* IPMDH as exemplified in any of Figures 1, 2 and 4.

12. A method according to claim 1, 2 or 11, further comprising formulating the compound as a fungicide.

13. A method of inhibiting the growth of a fungus by applying a compound identified by a method according to claim 1, 2 or 11 where the compound is selected from:
- phenyl 5-bromo-6-chloro-2-trifluoromethyl-benzimidazole-1-carboxylate;
- phenyl 4,5,6-tribromo-2-trifluoromethyl-benzimidazole-1-carboxylate; and
- 1,4-dimethylpentyl 4,5,6-tribromo-2-trifluoromethyl-benzimidazole-1-carboxylate.

## Patentansprüche

1. Verfahren zum Identifizieren von möglichen Fungiziden, bei dem man eine Verbindung in einem 3-Isopropylmalatdehydrogenase -(IPMDH-) Hemmtest testet, wobei es sich bei der IPMDH um die IPMDH aus *Septoria nodorum* gemäß einer der Abbildungen 1, 2 und 4 handelt und wobei eine meßbare Reduktion des Enzyms beobachtet wird, und die Verbindung wird anschließend (einem) traditionellen Test(s) unterzogen, um die fungizide Wirksamkeit in vivo zu bestätigen.

2. Verfahren nach Anspruch 1, bei dem man zur Erzeugung des Enzyms 3-Isopropylmalatdehydrogenase einen Vektor verwendet, wobei es sich bei dem Vektor um folgendes handelt:
(i) ein beliebiges Expressionssystem, das eine exprimierbare Form der DNA-Sequenz aus pSAL203(NCIMB 40917) gemäß Abbildung 1 aus einem cDNA-Klon aus *Septoria nodorum,* der für 3-Isopropylmalatdehydrogenase codiert, beinhaltet; oder
(ii) ein beliebiges Expressionssystem, das eine exprimierbare Form des 3-Isopropylmalatdehydrogenasegens aus *Septoria nodorum* gemäß Abbildung 4 beinhaltet.

3. Nichtpathogene, Leucin benötigende Mutante von *Septoria nodorum*, die wie in Abbildung 1 oder 4 dargestellt in dem 3-Isopropylmalatdehydrogenasegen disrumpiert ist.

4. Nichtpathogene, Leucin benötigende *Septoria nodorum*-Mutante nach Anspruch 3, die unter der Bezeichnung 40.15 bekannt ist und unter der Eingangsnummer IMI 378472 hinterlegt ist.

5. Aminosäuresequenz nach Abbildung 2, die sich von einem *Septoria nodorum*-Gen, das für das Enzym 3-Isopropylmalatdehydrogenase codiert, ableitet.

6. DNA-Sequenz aus pSAL203(NCIMB 40917) gemäß Abbildung 1 aus einem cDNA-Klon von *Septoria nodorum*, die für 3-Isopropylmalatdehydrogenase codiert.

7. DNA-Sequenzen mit so viel Ähnlichkeit zu der Sequenz in Anspruch 6, daß ein Polypeptid mit derselben Aminosäuresequenz wie in Anspruch 5 codiert wird und die sich von einem pflanzenpathogenen Pilz ableiten.

8. DNA-Sequenz des leuA-Gens von *Septoria nodorum* gemäß Abbildung 4, die für das genomische Äquivalent der Sequenz in Anspruch 7 codiert.

9. Plasmid pSAL203, erhältlich von Stamm pSAL203/JA221, der unter der Bezeichnung NCIMB 40917 hinterlegt ist, Plasmid pSAL204, erhältlich von Stamm pSAL204/JA221, der unter der Bezeichnung NCIMB 40918 hinterlegt ist, Plasmid pSAL205, erhältlich von Stamm pSAL205/XL1, der unter der Bezeichnung NCIMB 40919 hinterlegt ist, Plasmid pSAL206, erhältlich von Stamm pSAL206/XL1, der unter der Bezeichnung NCIMB 40920 hinterlegt ist oder Plasmid pSAL208, erhältlich von Stamm pSAL208/XL1, der unter der Bezeichnung NCIMB 40921 hinterlegt ist.

10. Expressionssystem wie in Anspruch 2 definiert.

11. Verfahren zum Screenen auf ein potentielles Fungizid, wobei das Verfahren umfaßt, daß man eine Verbindung, die *Septoria nodorum-IPMDH* gemäß einer der Abbildungen 1, 2 und 4 hemmt, identifiziert.

12. Verfahren nach Anspruch 1, 2 oder 11, das weiterhin beinhaltet, daß man die Verbindung als Fungizid formuliert.

13. Verfahren zur Hemmung des Wachstums eines Pilzes durch Ausbringen einer Verbindung, die nach einem Verfahren nach Anspruch 1, 2 oder 11 identifiziert wurde, wobei die Verbindung aus der Reihe
- Phenyl-5-brom-6-chlor-2-trifluormethylbenzimidazol-1-carboxylat,
- Phenyl-4,5,6-tribrom-2-trifluormethylbenzimidazol-1-carboxylat und
- 1,4-Dimethylpentyl-4,5,6-tribrom-2-trifluormethylbenzimidazol-1-carboxylat
stammt.

## Revendications

1. Méthode d'identification de fongicides potentiels, qui consiste à tester un composé lors d'un dosage d'inhibition de la 3-isopropylmalate déshydrogénase (IPMDH) où l'IPMDH est l'IPMDH de *Septoria nodorum* telle qu'illustrée dans l'une quelconque des figures 1, 2 et 4 et où une réduction d'enzyme mesurable est observée, le composé étant par la suite soumis à un ou plusieurs tests classiques pour confirmer l'activité fongicide *in vivo*.

2. Méthode selon la revendication 1, dans laquelle un vecteur est utilisé pour produire l'enzyme 3-isopropylmalate déshydrogénase, le vecteur tétant :
(i) un quelconque système d'expression qui inclut une forme exprimable de séquence d'ADN de pSAL203 (NCIMB 40917) telle qu'illustrée en figure 1 provenant d'un clone d'ADNc de *Septoria nodorum* qui code pour la 3-isopropylmalate déshydrogénase ; ou
(ii) un quelconque système d'expression qui inclut une forme exprimable du gène de la 3-isopropylmalate déshydrogénase de *Septoria nodorum* tel que présenté en figure 4.

3. Mutant de *Septoria nodorum* non pathogène et nécessitant de la leucine, dont le gène de la 3-isopropylmalate déshydrogénase tel qu'illustré en figure 1 ou 4 est disrupté.

4. Mutant de *Septoria nodorum* non pathogène et nécessitant de la leucine selon la revendication 3, connu sous le nom 40.15 et déposé sous le numéro d'accession IMI 378472.

5. Séquence d'acides aminés telle qu'illustrée en figure 2, dérivée d'un gène de *Septoria nodorum* codant pour l'enzyme 3-isopropylmalate déshydrogénase.

6. Séquence d'ADN de pSAL203 (NCIMB 40917) telle qu'illustrée en figure 1 provenant d'un clone d'ADNc de *Septoria nodorum* qui code pour la 3-isopropylmalate déshydrogénase.

7. Séquences d'ADN dont la similarité avec la séquence selon la revendication 6 est suffisante pour coder pour un polypeptide présentant la même séquence d'acides aminés que celle revendiquée dans la revendication 5 et dérivées d'un champignon pathogène de plante.

8. Séquence d'ADN du gène leuA de *Septoria nodorum* telle qu'illustrée en figure 4, codant pour l'équivalent génomique de la séquence selon la revendication 7.

9. Plasmide pSAL203 pouvant être obtenu à partir de la souche pSAL203/JA221 déposée sous le numéro NCIMB 40917, plasmide pSAL204 pouvant être obtenu à partir de la souche pSAL204/JA221 déposée sous le numéro NCIMB 40918, plasmide pSAL205 pouvant être obtenu à partir de la souche pSAL205/XL1 déposée sous le numéro NCIMB 40919, plasmide pSAL206 pouvant être obtenu à partir de la souche pSAL206/XL1 déposée sous le numéro NCIMB 40920 ou plasmide pSAL208 pouvant être obtenu à partir de la souche pSAL208/XL1 déposée sous le numéro NCIMB 40921.

10. Système d'expression tel que défini dans la revendication 2.

11. Méthode de criblage d'un fongicide potentiel, la méthode consistant à identifier un composé qui inhibe l'IPMDH de *Septoria nodorum* telle qu'illustrée dans l'une quelconque des figures 1, 2 et 4.

12. Méthode selon la revendication 1, 2 ou 11, consistant en outre à formuler le composé sous forme de fongicide.

13. Méthode permettant d'inhiber la croissance d'un champignon en appliquant un composé identifié par une méthode selon la revendication 1, 2 ou 11 où le composé est choisi parme :
- le 5-bromo-6-chloro-2-trifluorométhylbenzimidazole-1-carboxylate de phényle ;
- le 4,5,6-tribromo-2-trifluorométhylbenzimidazole-1-carboxylate de phényle et
- le 4,5,6-tribromo-2-trifluorométhylbenzimidazole-1-carboxylate de 1,4-diméthylpentyle.
